# EUROPÄISCHE PATENTANMELDUNG

(11) **EP 2 075 361 A1**
(43) Veröffentlichungstag der Anmeldung: **01.07.2009**
(21) Anmeldenummer: 08022315.9
(22) Anmeldetag: 23.12.2008
(51) Int. Cl.: D04B 1/28

(54) **Nahtloses Kompressionsgestrick und Verfahren zu dessen Herstellung**

(30) Priorität: 29.12.2007 DE 102007063148
(71) Anmelder: Medi GmbH & Co. KG, 95448 Bayreuth (DE)
(72) Erfinder: Weihermüller, Michael, Dr., 95448 Bayreuth (DE)
(74) Vertreter: Dorn, Dietmar

(57) **Zusammenfassung**

Nahtloses Kompressionsstrickteil, als Schlauchgestrick auf einer Flachstrickmaschine mit mindestens zwei Nadelbetten hergestellt, welches mit einem oder mehreren elastischen Strickfaden und einem oder mehreren elastischen Schussfaden ausgeführt ist, wobei das Kompressionsstrickteil mindestens aus zwei Schlauchgestricken besteht, die in ein Basisschlauchteil münden, wobei die Anzahl der Maschenstäbchen der Schlauchteile in der Summe höher der Anzahl der Maschenstäbchen in dem Basisschlauchteil ist.

## Beschreibung

Die Erfindung betrifft ein, als Schlauchgestrick, auf einer Flachstrickmaschine hergestelltes Kompressionsstrickteil, welches mit einem oder mehreren elastischen Strickfäden und wenigstens einem elastischen Schussfaden ausgeführt ist und zum Stützen und/oder für die Kompressionsbehandlung von Körperteilen wie Hand und Finger, Fuß und Zehen oder Rumpf und Armen bzw. Beinen verwendet werden kann, sowie ein Verfahren zur Herstellung solcher Strickteile.

Kompressionsstrickartikel werden in Therapien z.B. bei Venenerkrankungen oder bei der Nachversorgung operativer Eingriffe eingesetzt. Hierbei gibt es neben lange bekannten Kompressionsartikeln, wie Bandagen zum Stützen oder Sportbandagen, Stützstrümpfe oder auch Kompressionsstrümpfe auch Kompressionsstrickteile als Handschuh oder Zehensocken um auch diese Bereiche und insbesondere die Finger oder die Zehen einer Kompressionsbehandlung zu unterziehen.
Während einfachere Formen von Strickteilen, insbesondere nahtlose Schlauchgestricke vielfältig auf Rundstrickmaschinen hergestellt werden hat sich für Gestricke mit höheren Anforderungen an die Passform und insbesondere bei der Anpassung an kompliziertere Formen die Herstellung auf Flachstrickmaschinen etabliert.

Durch die Möglichkeit der Herstellung vom kompressiven Strickteilen mittels einer Flachstrickmaschine, auf welcher ein nadelgenaues Mindern und Zunehmen von Maschen möglich ist, lassen sich auch komplexere Formen, wie etwa einen Handschuh als flächiges Gebilde der Handform anpassen.
Auf Flachstrickmaschinen lassen sich solche Strickteile auch als einteilige Schlauchgestricke in einem Strickprozess herstellen, wie z.B. in der DE 40 06 877 A1 beschrieben wird. An einen Pullover werden hier direkt beim Stricken die Armteile angestrickt und der Strickprozess einer geforderten Passform angepasst.
In der beschriebenen Art und Weise der Minderung von Maschen während des Strickens ist die Anzahl von Maschenstäbchen die in einer Maschenreihe gemindert werden können, durch die Elastizität des Strickfadens, der Länge der Platinenmasche und der Maschengröße begrenzt.

Auch die DE 196 52 612 A1 offenbart ein schlauchförmiges Gestrick, hergestellt auf einer Zweibett-Flachstrickmaschine und ein Verfahren zur Maschenzunahme in einem solchen Gestrick während des Strickvorgangs.

In der EP 1 679 012 A1 wird ein solches Strickteil beschrieben, welches einen Handschuh mit hoher Stützkraft bildet und aus elastischem Strickgarn besteht und welches zusätzlich einen elastischen Schuss- oder Einlegefaden aufweisen kann, was für Kompressionsgestricke bzw. Gestricke mit kompressiver Wirkung sehr wichtig ist.
Bei einer solchen Ausführungsform wird ein kompressives Strickteil geschaffen, welches vollständig nahtlos ist und auch die Herstellung komplexerer Formen, wie z.B. einen Handschuh erlaubt.
Durch den eingebrachten Schussfaden und insbesondere dessen Spannung, können die Elastizitätseigenschaften der Strickware gut gesteuert werden.

Bei der Herstellung solcher nahtloser, kompressiver Strickteile, bevorzugt unter Verwendung einer 1:1 Bindung, sind aber einige Problembereiche und damit verbundene Unzulänglichkeiten vorhanden, was beispielhaft an einem Handschuh näher betrachtet wird.

Betrachtet man eine Hand, so ist die Summe der Umfänge der einzelnen Finger größer als der Umfang des Handballens. In einem nach dem Stand der Technik beschriebenen Strickteil bzw. kompessiven Strickteil, ist diesem Umstand aber nicht ausreichend Rechnung getragen, da bei gleicher Nadelzahl in der Breite die Kompression an den Fingern zu hoch wird, bzw. nicht der gewünschte oder erforderliche Druckverlauf erzielt wird.

Nach dem Stand der Technik muss daher vor dem Übergang von den einzelnen Schlauchteilen in ein Basisschlauchteil die Summe der Durchmesser der einzelnen Schlauchteile an den Durchmesser des Basisschlauchteils angepasst werden um den Übergang zu vollziehen.
In den bekannten Ausführungen und Verfahren werden dadurch z.B. bei einem Handschuh die Durchmesser der Schlauchteile der einzelnen Finger bei Annäherung an den Übergangsbereich zum Basisteil gemindert. Dadurch wird erreicht, dass beim Übergang zum Basisschlauchteil die Summe der Durchmesser aller Schlauchteile gleich dem Durchmesser des Basisschlauchteiles in welches der Übergang erfolgt, ist, was nach dem Stand der Technik nötig ist.
Verständlicher Weise gelangt man hier sehr schnell an Grenzen für die Ausführbarkeit von Handschuhen für die Behandlung von Patienten mit stark angeschwollenen Fingern. Es wäre grundsätzlich kein Problem, die Schlauchteile für die Finger angepasst zu stricken, was aber den Unterschied der Durchmesser der einzelnen Schlauchteile bezogen auf den Durchmesser des Schlauchteils der Hand, noch weiter auseinanderklaffen lässt.
Hier muss dann im Bereich vor dem Übergang entweder der Durchmesser der einzelnen Schlauchteile stark gemindert werden oder grundsätzlich in Kauf genommen werden, dass die einzelnen Schlauchteile (Fingerteile) enger als optimal erforderlich ausgeführt sind. Dadurch ergibt sich zwangsweise ein nicht optimaler Druckverlauf, was z.B. für einen medizinischen Handschuh sehr negativ ist. Man erhält entweder einen richtigen Druckverlauf in den Bereichen der Finger und dadurch einen nicht passenden, zu niedrigen Druckverlauf in dem Bereich der Hand und des Handgelenkes oder wie bei den meisten bekannten Strickteilen gemäß des Stands der Technik einen Kompressionshandschuh mit zu hohem Druck in den Fingern.
Durch die aufgezeigten Grenzen können z.B. auch Patienten mit zu stark angeschwollenen Fingern nicht behandelt werden.

Aufgabe der vorliegenden Erfindung ist es, ein nahtloses Kompressionsstrickteil, als Schlauchstrickteil zum Stützen oder Komprimieren von Körperteilen zu schaffen, welches einen hohen Tragekomfort bietet und eine optimierte Anpassung des erforderlichen Kompressionsverlaufs ermöglicht.

Diese Aufgabe wird mit den Merkmalen des kennzeichnenden Teils des Anspruchs 1 und 12 bis 15 gelöst. Fortbildungen und vorteilhafte Ausführungen der Erfindung sind in den weiteren Ansprüchen umfasst.

Erfindungsgemäß ist ein nahtloses Kompressionsstrickteil, als Schlauchgestrick auf einer Flachstrickmaschine mit mindestens zwei Nadelbetten hergestellt, welches mit einem oder mehreren elastischen Strickfaden und einem oder mehreren elastischen Schussfaden ausgeführt ist, wobei das Kompressionsstrickteil mindestens aus zwei Schlauchgestricken besteht, die in ein Basisschlauchteil münden, wobei die Anzahl der Maschenstäbchen der Schlauchteile in der Summe höher der Anzahl der Maschenstäbchen in dem Basisschlauchteil ist.

Durch eine entsprechende Zunahme beim Stricken könnte grundsätzlich auch erreicht werden, dass die Anzahl der Maschenstäbchen in der Summe aller Schlauchteile niedriger ist als die Anzahl der Maschenstäbchen in dem Basisschlauchteil, was aber die Ausnahme sein wird.

Eine dahingehende Anwendung wäre z.B. ein kompressiver Fingerhandschuh, an welchem fünf Finger-Schlauchgestricke in ein Basisschlauchgestrick um die Hand münden. Man kann den Handschuh natürlich auch als Basisschlauchgestrick um die Hand beschreiben, von welchem fünf Finger-Schlauchgestricke abgehen, dies bedarf lediglich einer Anpassung im Strickprozess, bzw. der Reihenfolge des Strickens.

Da die sich ergebende Summe aller Umfänge von fünf Fingern größer ist als der Umfang des Handballens ist die erstgenannte Ausgestaltung, in welcher die Anzahl der Maschenstäbchen der Schlauchteile in der Summe höher der Anzahl der Maschenstäbchen in dem Basisschlauchteil ist, die bevorzugte Ausführung.

Durch diese Möglichkeit der Anpassung der Umfänge der einzelnen Schlauchteile, ungleich zum Umfang des Basisschlauchteils, wird der am Stand der Technik bekannte Mangel überwunden. Bisherige Strickteile hatten durch gleichbleibende Maschenstäbchen einen gleichen Umfang in der Summe der Umfänge der Schlauchteile und des Basisschlauchteils, was zu einer, in der Regel, höheren Kompression in den einzelnen Schlauchteilen führte.
Mit der Ausgestaltung eines erfindungsgemäßen kompressiven Strickteils kann somit ein Handschuh geschaffen werden, welcher einen gleichen Druck in den Fingern und im Bereich der übrigen Hand aufweist, wobei der sich einzustellende Druck in den Schlauchteilen und dem Basis-Schlauchteil individuell gesteuert werden kann.

Das erfindungsgemäße, nahtlose Kompressionsstrickteil ist bevorzugt ein Fingerhandschuh oder eine Zehensocke, kann aber auch als ein anderes Kompressionsstrickteil ausgeführt sein, wie ein Oberteil, Body oder Shirt, oder auch als eine Hose, Pants oder Leggins.

Ein weiterer, wesentlicher Vorteil gegenüber dem Stand der Technik ist die Ausgestaltung eines solchen Strickteils, in welchem die einzelnen Strickteile über ihre jeweilige Länge einen individuellen Durchmesser haben, welcher sich auch im Übergangsbereich zum Basisgestrick nicht allmählich ändert, wie dies aus dem Stand der Technik bekannt ist.

Ein erfindungsgemäßes Strickteil ist bevorzugt mit einem Übergang, bzw. einer Einmündung von einem Schlauchteil in ein Basis-Schlauchteil oder umgekehrt, in einer Maschenreihe ausgeführt. Das Schlauchteil kann damit den individuell gewünschten Querschnitt vom Übergangspunkt bis zu seinem Ende aufweisen.

Die Überganspunkte von einem Basisschlauchteil in ein oder mehrere Schlauchteile können frei in Maschenreihen gewählt werden und erfolgen für jedes abgehende Schlauchteil bevorzugt in einer Maschenreihe. Damit ist es gemäß der Erfindung möglich jedes Schlauchteil einzeln, mehrere oder alle Schlauchteile gleichzeitig in einer Maschenreihe in das, bzw. aus dem Basisschlauchteil münden zu lassen. Damit können unterschiedliche Höhen der Abgänge von Fingerschlauchteilen individuell ausgeführt werden.

Die Ausgestaltung eines erfindungsgemäßen, nahtlosen Kompressionsstrickteils erlaubt den Übergang von einem Schlauchteil zu einem Basis-schlauchteil oder umgekehrt in der Zunahme oder Abnahme von beliebig vielen Maschenstäbchen, was lediglich von dem maximalen Versatzweg der Nadelbetten der Flachstrickmaschine begrenzt ist.

Eine nach dem Stand der Technik übliche Begrenzung der Zunahme oder Abnahme von einem Maschenstäbchen pro Maschenreihe und der sich dadurch ergebenden stetigen Veränderung der Durchmesser der einzelnen Schlauchteile vor dem Übergang in ein Basisschlauchteil entfällt.

Nachfolgend wird die Herstellung eines erfindungsgemäßen nahtlosen Kompressionsstrickteils am Beispiel eines Fingerhandschuhes beschrieben, in welchem Zusammenhang auch die Fadenverlauf-Abbildungen stehen.

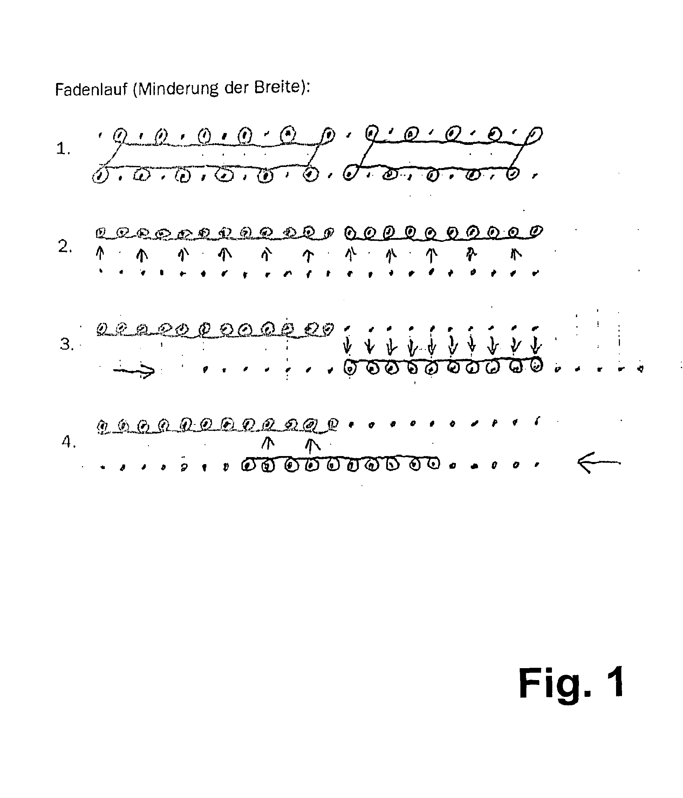

- Fig. 1: zeigt dabei den Ablauf mit der Minderung der Breite

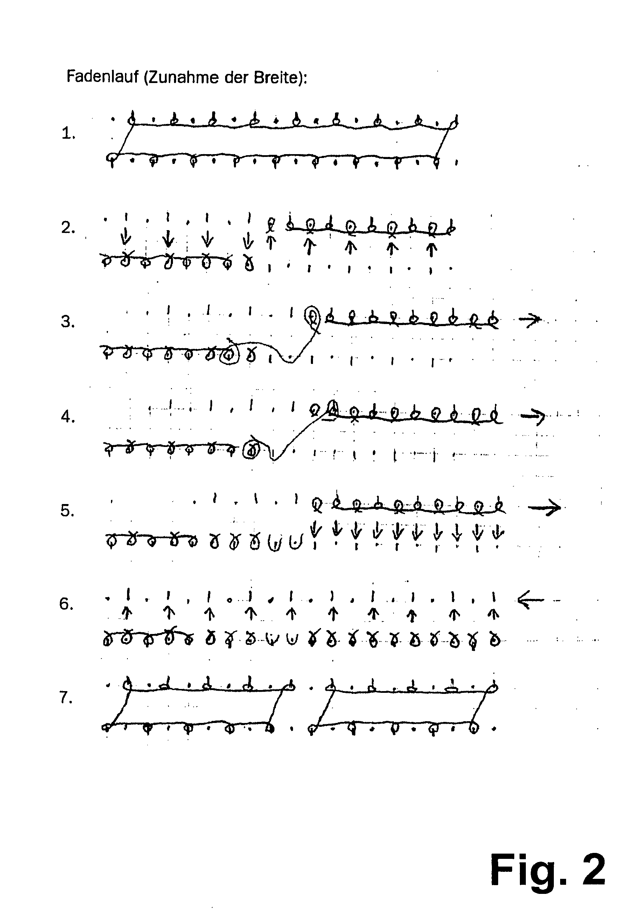

- Fig. 2: zeigt den Ablauf bei Zunahme der Breite

Das Grundgestrick ist eine Röhre mit eingelegtem Schussfaden (Schlauch auf 1:1 Basis). d.h. nur jede zweite Nadel strickt auf dem vorderen und dem hinteren Nadelbett. Die Herstellung so eines Stütz- bzw. Kompressionsgestricks (Maschen verteilen, bzw. auf leere Nadeln aufnehmen, Schussfaden einlegen, abstricken, Maschen sammeln) ist allgemein bei Fachleuten bekannt.

Das Problem ergibt sich am Übergang der "Fingerröhren" zur "Handballenröhre". Entweder ist die Kompression in den "Fingerröhren" zu hoch und in der "Handballenröhre" korrekt, oder in den "Fingerröhren" korrekt und in der Handballenröhre zu niedrig. Das Mindern, bzw. das Zunehmen von Maschenstäbchen (Maschenanzahl in der Breite) im Übergangsbereich bedarf nach dem Stand der Technik mehrere Maschenreihen (Maschenanzahl in der Höhe) und ist daher als Lösung nicht geeignet.

Zuerst werden die "Fingerröhren" nacheinander, nebeneinander, oder gleichzeitig nebeneinander, in der benötigten Breite und Form gestrickt. Da aber auf dem vorderen Nadelbett, sowie auf dem hinteren Nadelbett nur jede zweite Nadel belegt ist, können alle vorderen Maschen neben den hinteren Maschen, also auf Lücke, auf dem hinteren Nadelbett gesammelt (umgehängt) werden. Dann wird das vordere Nadelbett versetzt und die Maschen der ersten Röhre komplett auf das vordere Nadelbett umgehängt. In wie weit das Nadelbett versetzt wird, hängt von der gewünschten Überlappung der ersten Röhre zur zweiten Röhre ab, d. h. der Verringerung des Gesamtumfangs von Röhre 1 mit Röhre 2.
Nach dem Rückversetzen des vorderen Nadelbetts werden die überlappenden hinteren Nadeln der ersten Röhre (alle Maschenstäbchen vorne gesammelt) auf die überlappenden vorderen Nadeln der zweiten Röhre (alle Maschenstäbchen hinten gesammelt) gehängt und im Bereich der Überlappung abgekettelt.

Abhängig von der Position der Fadenführer werden die restlichen vorderen Maschen der zweiten Röhre nach vorne verteilt und die restlichen hinteren Maschen der ersten Röhre nach hinten, bei Bedarf werden auch die Maschen der restlichen "Fingerröhren" verteilt. Dieser Gesamtvorgang kann dann so oft wiederholt werden, bis alle Fingerröhren abgearbeitet worden sind, oder die neu gebildete Röhre wird weitergestrickt und die restlichen Fingerröhren werden nach Muster in der oben aufgeführten Weise eingefügt.

Es ist auch möglich alle Maschen auf dem vorderen Nadelbett zu sammeln, dann das hintere Nadelbett zu versetzen und die Maschen der ersten Röhre komplett auf das hinteren Nadelbett um zuhängen. Nach dem Rückversetzen des hinteren Nadelbetts werden die überlappenden vorderen Nadeln der ersten Röhre (alle Mst hinten gesammelt) auf die hinteren Nadeln der zweiten Röhre (alle Mst vorne gesammelt) gehängt und im Bereich der Überlappung abgekettelt, usw.

Auch eine Kombination der beiden Wege ist möglich. Auf diese Weise ist jeder benötigte Fingerumfang darstellbar, auch der durch Lympherkrankung angeschwollener Glieder. Eine anatomisch korrekte Platzierung der "Daumenröhre" ist auf diese Weise auch gewährleistet.

Auch eine Umkehrung dieser Vorgehensweise ist möglich, das heißt, das Gestrick beginnt mit der "Handröhre" um dann in "Fingerröhren" überzugehen, deren Umfang sofort die erforderliche Anzahl von Maschenstäbchen hat, ohne die sonst nötige Zunahme über mehrere Maschenreihen und die dadurch bedingte schrittweise Zunahme des Umfangs.
Zuerst wird die "Handröhre" in der benötigten Breite in der oben ausgeführten Bindung gestrickt. Als nächstes werden die Maschen des vorderen Nadelbetts über der gesamten Breite abgestrickt. Dann werden die Maschen des hinteren Nadelbetts, die nicht für die "Fingerröhre" benötigt werden, das heißt, die "Fingerröhre" beginnt sofort mit dem für die Kompression korrektem Umfang, in Lücke auf das vordere Nadelbett umgehängt. Danach werden die hinteren Maschen der "Fingerröhre" abgestrickt und die vorderen Maschen der "Fingerröhre" in Lücke auf das hintere Nadelbett gehängt.
Nun wird das hintere Nadelbett um 2 Maschen versetzt, so dass sich die Fingerröhre von der "Handröhre" entfernt. Als nächstes wird die Lücke zwischen "Handröhre" und "Fingerröhre" gestopft, dies kann in unterschiedliche Weise geschehen, als Beispiel in nachfolgender Ausführung. In einem Schlittenhub wird die innerste, vorderste Masche der "Handröhre" auf dem vorderen Nadelbett abgestrickt, in die zweite leere Nadel, zur "Fingerröhre" hin im vorderen Nadelbett, der Faden eingelegt (Fang) und die innerste, vordere Masche der "Fingerröhre" auf dem hinteren Nadelbett abgestrickt. Im Schlittenhub zurück wird die innerste, hintere Masche der "Fingerröhre" auf dem hinteren Nadelbett abgestrickt, in die letzte leere Nadel, zur "Handröhre" hin im vorderen Nadelbett, der Faden eingelegt (Fang) und die innerste, hintere Masche der "Handröhre" im vorderen Nadelbett abgestrickt. Danach wird das hintere Nadelbett, gemäß der oberen Ausführung, um weitere 2 Nadeln versetzt, das Stopfen der Lücke verschiebt sich gemäß dem Versatz. Der Ablauf dieser gesamten Vorgänge wird solange wiederholt, bis die benötigte Breite der "Handröhre" erreicht ist.
Wenn die benötigte Breite der "Handröhre" erreicht ist, werden alle Maschen der "Fingerröhre" auf das vordere Nadelbett übertragen und das hintere Nadelbett in Grundposition zurück gefahren. Abhängig von der Position der Fadenführer werden die hinteren Maschen der "Handröhre" , die durch das "Stopfen" neu gebildeten Maschen für das hintere Nadelbett und die hinteren Maschen der "Fingerröhre" auf das hintere Nadelbett zurück gehängt. Dieser Gesamtvorgang kann dann so oft wiederholt werden, bis alle "Fingerröhren" fertig sind, oder die neu gebildeten Röhren werden einzeln oder gemeinsam weitergestrickt und die restlichen "Fingerröhren" werden nach Muster in der oben aufgeführten Weise aus gedeckt.
Es ist auch möglich, auf dieser Weise, die "Handröhre" aus der "Fingerröhre" heraus zu führen, bzw. beide Wege zu kombinieren.
Bei Flachstrickmaschinen mit Versatzeinrichtung im vorderen Nadelbett ist die gleiche Technik möglich, in dem oben ausgeführten Ablauf ist dann vorne mit hinten und hinten mit vorne zu tauschen. Sollte die Flachstrickmaschine über die bauliche Möglichkeit verfügen, ist auch eine Mixtur von beiden Wegen möglich.
Die gleiche Vorgehensweise gilt bei Zehenkappen, Hosen (Beinumfänge zu Hüftumfang) und sonstigen gestrickten Röhren deren Summe der Einzelumfänge größer ist als der Gesamtumfang der "Endröhre" , bzw. die "Anfangsröhre" einen kleineren Umfang hat als die aus ihr hervorgehenden "Röhren" deren Summe der Einzelumfänge größer ist als der Gesamtumfang der "Anfangsröhre"

Nachdem bevorzugte Ausführungen der Erfindung in Bezug auf die beiliegenden Zeichnungen beschrieben wurden, ist festzuhalten, dass die Erfindung nicht auf diese genauen Ausführungen und Anwendungsfälle beschränkt ist und dass verschiedene Änderungen und Modifizierungen daran von einem Fachmann ausgeführt werden können, ohne dass vom Umfang der Erfindung, wie er in den beiliegenden Ansprüchen definiert ist abgewichen wird.

## Patentansprüche

1. Nahtloses Kompressionsstrickteil, als Schlauchgestrick auf einer Flachstrickmaschine mit mindestens zwei Nadelbetten hergestellt, welches mit einem oder mehreren elastischen Strickfaden und einem oder mehreren elastischen Schussfaden ausgeführt ist,
**dadurch gekennzeichnet,**
**dass** das Kompressionsstrickteil mindestens aus zwei Schlauchgestricken besteht, die in ein Basisschlauchteil münden, wobei die Anzahl der Maschenstäbchen der Schlauchteile in der Summe höher der Anzahl der Maschenstäbchen in dem Basisschlauchteil ist.

2. Nahtloses Kompressionsstrickteil nach Anspruch 1
**dadurch gekennzeichnet,**
**dass** von einem Basisschlauchteil wenigstens zwei Schlauchteile abgehen, wobei die Anzahl der Maschenstäbchen der Schlauchteile in der Summe höher der Anzahl der Maschenstäbchen in dem Basisschlauchteil ist.

3. Nahtloses Kompressionsstrickteil nach Anspruch 1 oder 2
**dadurch gekennzeichnet,**
**dass** die Anzahl der Maschenstäbchen in der Summe aller Schlauchteile niedriger ist als die Anzahl der Maschenstäbchen in dem Basisschlauchteil.

4. Nahtloses Kompressionsstrickteil nach Anspruch 1
**dadurch gekennzeichnet,**
**dass** mindestens zwei Schlauchteile in ein Basisschlauchteil münden.

5. Nahtloses Kompressionsstrickteil nach Anspruch 1
**dadurch gekennzeichnet,**
**dass** fünf, oder mehr, Schlauchteile in ein Basisschlauchteil münden.

6. Nahtloses Kompressionsstrickteil nach Anspruch 1 oder 2
**dadurch gekennzeichnet,**
**dass** der Durchmesser der Schlauchteile beim Einmünden in das Basis-schlauchteil unverändert ist.

7. Nahtloses Kompressionsstrickteil nach Anspruch 1 oder 2
**dadurch gekennzeichnet,**
**dass** die Einmündung der Schlauchteile in das Basisschlauchteil in einer Maschenreihe erfolgt.

8. Nahtloses Kompressionsstrickteil nach Anspruch 1 oder 2
**dadurch gekennzeichnet,**
**dass** die Minderung der Schlauchteile nur durch den maximalen Versatzweg der Nadelbetten begrenzt ist.

9. Nahtloses Kompressionsstrickteil nach Anspruch 1 oder 2
**dadurch gekennzeichnet,**
**dass** das Kompressionsteil eine Hose oder ein Oberteil ist, bei welchem von einem Basisschlauteich zwei weitere Schlauchteile abgehen.

10. Nahtloses Kompressionsstrickteil nach Anspruch 1 oder 2
**dadurch gekennzeichnet,**
**dass** das Kompressionsteil ein Handschuh oder eine Zehensocke ist, bei welchem von einem Basisschlauteich fünf weitere Schlauchteile abgehen.

11. Nahtloses Kompressionsstrickteil nach Anspruch 1 oder 2
**dadurch gekennzeichnet,**
**dass** das Kompressionsstrickteil ein medizinisches Kompressionsstrickteil ist und die einzelnen Schlauchteile sowie das Basisschlauchteil, individuell und maschengenau gestrickt, ausgeführt sind.

12. Verfahren zur Herstellung eines nahtlosen Kompressionsstrickteils als Schlauchgestrick auf einer Zweibett-Flachstrickmaschine hergestellt, welches mit einem oder mehreren elastischen Strickfaden und einem oder mehreren elastischen Schussfaden ausgeführt ist,
**gekennzeichnet durch** folgende Schritte,
- Stricken der Schlauchteile in der benötigten Breite und Form mit Belegung jeder zweiten Nadel auf dem vorderen und hinteren Nadelbett
- Sammeln bzw. Umhängen aller Maschen auf das hintere Nadelbett
- Versetzen des vorderen Nadelbettes um einen geeigneten Wert
- Maschen des ersten Schlauches komplett auf das vordere Nadelbett umhängen.
- Rückversetzen des vorderen Nadelbetts
- Umhängen der überlappenden hinteren Nadeln des ersten Schlauches auf die überlappenden vorderen Nadeln des zweiten Schlauches.
- Abketteln der zusammengehängten Maschen (hintere Maschen des ersten Schlauchs und vordere Maschen des zweiten Schlauchs) im Bereich der Überlappung.
- Verteilung der restlichen vorderen Maschen des zweiten Schlauches nach vorne und der restlichen hinteren Maschen des ersten Schlauches nach hinten, in Abhängigkeit der Position des Fadenführers.
- Verteilung der Maschen der restlichen Schläuche nach Bedarf
- Wiederholung der Vorgänge bis alle zusätzlichen Schlauchteile abgearbeitet worden sind. Oder Abstricken des neuen, aus dem ersten und dem zweiten Schlauch gebildeten, Schlauchs und eine spätere Wiederholung der Vorgänge bis alle zusätzlichen Schlauchteile abgearbeitet worden sind.

13. Verfahren zur Herstellung eines nahtlosen Kompressionsstrickteils als Schlauchgestrick auf einer Zweibett-Flachstrickmaschine hergestellt, welches mit einem oder mehreren elastischen Strickfaden und einem oder mehreren elastischen Schussfaden ausgeführt ist,
**gekennzeichnet durch** folgende Schritte,
- Stricken der Schlauchteile in der benötigten Breite und Form mit Belegung jeder zweiten Nadel auf dem vorderen und hinteren Nadelbett
- Sammeln bzw. Umhängen aller Maschen auf das vordere Nadelbett Versetzen des hinteren Nadelbettes um einen geeigneten Wert
- Maschen des ersten Schlauches komplett auf das hintere Nadelbett umhängen.
- Rückversetzen des hintere Nadelbetts
- Umhängen der überlappenden vorderen Nadeln des ersten Schlauches auf die überlappenden hinteren Nadeln des zweiten Schlauches.
- Abketteln der zusammengehängten Maschen (vordere Maschen des ersten Schlauchs und hintere Maschen des zweiten Schlauchs) im Bereich der Überlappung.
- Verteilung der restlichen hinteren Maschen des zweiten Schlauches nach hinten und der restlichen vorderen Maschen des ersten Schlauches nach vorne, in Abhängigkeit der Position des Fadenführers.
- Verteilung der der Maschen der restlichen Schläuche nach Bedarf
- Wiederholung der Vorgänge bis alle zusätzlichen Schlauchteile abgearbeitet worden sind. Oder Abstricken des neuen, aus dem ersten und dem zweiten Schlauch gebildeten, Schlauchs und eine spätere Wiederholung der Vorgänge bis alle zusätzlichen Schlauchteile abgearbeitet worden sind.

14. Verfahren zur Herstellung eines nahtlosen Kompressionsstrickteils als Schlauchgestrick auf einer Flachstrickmaschine mit Umhängeplatinen hergestellt, welches mit einem oder mehreren elastischen Strickfaden und einem oder mehreren elastischen Schussfaden ausgeführt ist, **gekennzeichnet durch** folgende Schritte,
- Stricken der Schlauchteile in der benötigten Breite und Form mit Belegung jeder zweiten Nadel auf dem vorderen und hinteren Nadelbett
- Sammeln bzw. Umhängen aller Maschen auf das hintere Nadelbett
- Maschen des ersten Schlauches komplett auf die Umhängeplatinen umhängen.
- Versetzen der Umhängeplatinen und umhängen der Maschen des ersten Schlauchs auf die Nadeln des vorderen Nadelbetts.
- Umhängen der überlappenden hinteren Nadeln des ersten Schlauches auf die überlappenden vorderen Nadeln des zweiten Schlauches.
- Abketteln der zusammengehängten Maschen (hintere Maschen des ersten Schlauchs und vordere Maschen des zweiten Schlauchs) im Bereich der Überlappung.
- Verteilung der restlichen vorderen Maschen des zweiten Schlauches nach vorne und der restlichen hinteren Maschen des ersten Schlauches nach hinten, in Abhängigkeit der Position des Fadenführers.
- Verteilung der der Maschen der restlichen Schläuche nach Bedarf
- Wiederholung der Vorgänge bis alle zusätzlichen Schlauchteile abgearbeitet worden sind. Oder Abstricken des neuen, aus dem ersten und dem zweiten Schlauch gebildeten, Schlauchs und eine spätere Wiederholung der Vorgänge bis alle zusätzlichen Schlauchteile abgearbeitet worden sind

15. Verfahren zur Herstellung eines nahtlosen Kompressionsstrickteils als Schlauchgestrick auf einer Flachstrickmaschine mit Umhängeplatinen hergestellt, welches mit einem oder mehreren elastischen Strickfaden und einem oder mehreren elastischen Schussfaden ausgeführt ist, **gekennzeichnet durch** folgende Schritte,
- Stricken der Schlauchteile in der benötigten Breite und Form mit Belegung jeder zweiten Nadel auf dem vorderen und hinteren Nadelbett
- Sammeln bzw. Umhängen aller Maschen auf das vordere Nadelbett
- Maschen des ersten Schlauches komplett auf die Umhängeplatinen umhängen.
- Versetzen der Umhängeplatinen und umhängen der Maschen des ersten Schlauchs auf die Nadeln des hinteren Nadelbetts.
- Umhängen der überlappenden vorderen Nadeln des ersten Schlauches auf die überlappenden hinteren Nadeln des zweiten Schlauches.
- Abketteln der zusammengehängten Maschen (vordere Maschen des ersten Schlauchs und hintere Maschen des zweiten Schlauchs) im Bereich der Überlappung.
- Verteilung der restlichen hinteren Maschen des zweiten Schlauches nach hinten und der restlichen vorderen Maschen des ersten Schlauches nach vorne, in Abhängigkeit der Position des Fadenführers.
- Verteilung der der Maschen der restlichen Schläuche nach Bedarf
- Wiederholung der Vorgänge bis alle zusätzlichen Schlauchteile abgearbeitet worden sind. Oder Abstricken des neuen, aus dem ersten und dem zweiten Schlauch gebildeten, Schlauchs und eine spätere Wiederholung der Vorgänge bis alle zusätzlichen Schlauchteile abgearbeitet worden sind
